# EUROPEAN PATENT SPECIFICATION

(11) **EP 1 588 729 B1**
(45) Date of publication and mention of the grant of the patent: **05.11.2008**
(21) Application number: 05252088.9
(22) Date of filing: 01.04.2005
(51) Int. Cl.: A61M 5/145

(54) **Extrusion-type liquid delivery apparatus**
Extrusionsartiges Flüssigkeitsabgabesystem
Système de délivrance de liquide de type extrusion

(30) Priority: 02.04.2004 JP 2004110047; 01.11.2004 JP 2004317991
(43) Date of publication of application: 26.10.2005
(73) Proprietor: JAPAN SERVO CO., LTD., Chiyoda-Ku Tokyo (JP)
(72) Inventor: Sasaki, Naotaka, Kiryu-shi Gunma-ken (JP); Kawamata, Shunichi, Kiryu-shi Gunma-ken (JP); Sugaya, Kenji, Kiryu-shi Gunma-ken (JP); Kagawa, Yoshihisa, Kiryu-shi Gunma-ken (JP); Ito, Hideaki, Kiryu-shi Gunma-ken (JP)
(74) Representative: Gill, Stephen Charles

(56) References cited:
- EP-A- 0 323 321
- EP-A- 1 110 569
- WO-A-03/103749
- WO-A-20/04014465
- US-B1- 6 428 509

## Description

### BACKGROUND OF THE INVENTION

### 1. Field of the Invention

The present invention relates to an extrusion-type liquid delivery apparatus to deliver a liquid contained beforehand in a container with a plunger, such as an extrusion-type liquid delivery apparatus for medical use.

### 2. Description of the Related Art

Typically, the extrusion-type liquid delivery apparatus, which pushes a liquid contained in a container out with a plunger along the inner wall of the container, employs the technique of pushing the collar provided at one end of the plunger to move the plunger and thereby to push out a liquid contained in the container. In this case, a liquid delivery portion is provided on the side opposite to the side where the plunger is inserted in the liquid-filled container, and the liquid delivery portion has a small hole for delivering the liquid. In many cases, a tube composing a liquid delivery channel is connected to this liquid delivery portion, through which the liquid can be delivered to a desired place by a push motion of the plunger. If precise control of liquiddelivery is not required, the plunger may be pushed manually. However, when liquid delivery is performed at a constant rate over a long period of time, the extrusion-type liquid delivery apparatus is used.

The conventional extrusion-type liquid delivery apparatus has a gripping device to grip the collar provided at one end of the plunger.

With the conventional extrusion-type liquid delivery apparatus, however, gripping of the collar of the plunger is done manually by the operator. Because of this, it is difficult for one operator to load a drug-filled container on a plurality of extrusion-type liquid delivery apparatuses one by one and to start a liquid delivery, especially in emergencies .

WO 2004/014465 discloses a medicine liquid injection device wherein, when medicine is injected into a subject from one of two syringes, a syringe tube connected to the other syringe is closed. Thus, medicine injected into the subject from one syringe cannot flow back into the other syringe.

WO 03/103749 discloses a syringe plunger driver system with a pair of asymmetric plunger retainer arms pivotally mounted to a plunger driver.

### SUMMARY OF THE INVENTION

An object of the present invention is to provide an extrusion-type liquid delivery apparatus capable of automatically gripping the collar of the plunger.

Accordingly, the present invention provides an extrusion-type liquid delivery apparatus according to claim 1.

In this extrusion-type liquid delivery apparatus, it is possible to automatically rotate the bent hooks to grip the collar of the plunger with the hooks simply by moving the plunger mover, and therefore the operator can load, with a plurality of delivery apparatuses, a liquid-filled container that contains a liquid and grip the plunger in a short period of time. Moreover, since an additional special actuator for automatic gripping is not required, this apparatus can be of simpler construction and can be manufactured at lower cost.

### BRIEF DESCRIPTION OF THE DRAWINGS

Fig. 1 shows an extrusion-type liquid delivery apparatus according to the present invention;
Fig. 2 shows a configuration of a clamp, a container, and a liquid delivery channel of the extrusion-type liquid delivery apparatus shown in Fig. 1;
Fig. 3 is a block diagram showing a control device of the extrusion-type liquid delivery apparatus shown in Fig. 1; and
Fig. 4 through Fig. 6 are schematic diagrams illustrating the operation of the extrusion-type liquid delivery apparatus shown in Fig. 1.

### DETAILED DESCRIPTION OF THE INVENTION

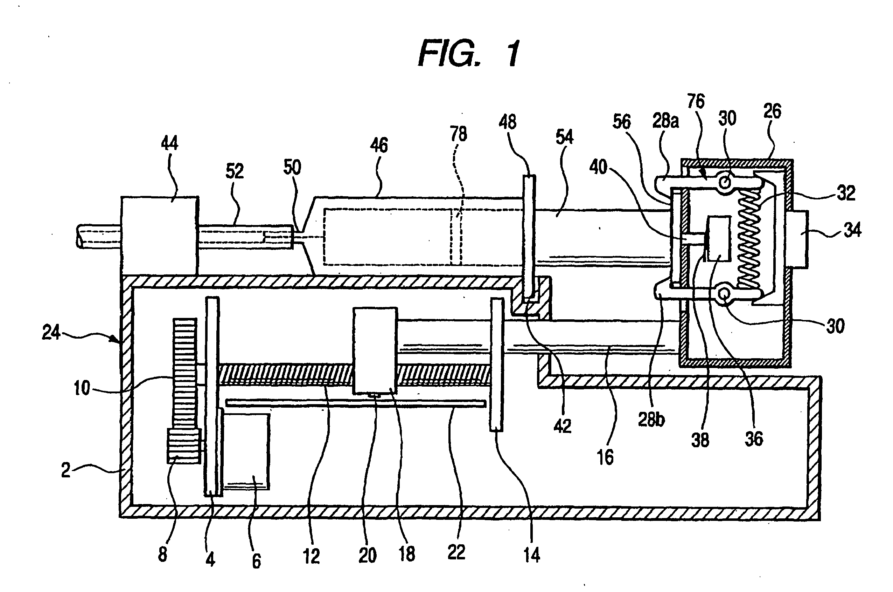

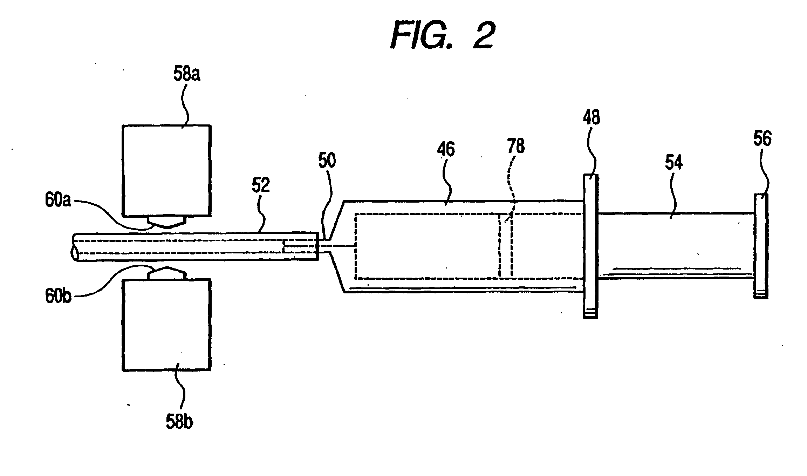

Referring to Fig. 1 and Fig. 2, an extrusion-type liquid delivery apparatus of the present invention will be described. Support frames 4 and 14 are fixed to a frame 2. A drive motor 6 is mounted on the support frame 4, and a pinion gear 8 is mounted on the output shaft of the drive motor 6. The support frames 4 and 14 rotatably support a lead screw 12. A driven gear 10 is mounted at one end of the lead screw 12, and the driven gear 10 engages with the pinion gear 8. A guide rod 16 is movably supported by the frame 2 and the support frame 14. A carriage 18 is mounted at one end of the guide rod 16, and the carriage 18 is provided with a nut (not shown) that engages with the lead screw 12. The carriage 18 is also provided with an optical sensor 20. An linear encoder 22 is fixed to the frame 2, the linear encoder 22 is parallel with the lead screw 12. That is, the carriage 18 is provided with the optical sensor 20, and the linear encoder 22 is disposed to face the optical sensor 20. A slit is formed on the linear encoder 22, thereby allowing the current position of the carriage 18 to be located based on an encoder signal from the optical sensor 20. Thus, the drive motor 6, the lead screw 12, the carriage 18, etc. compose a plunger mover driver 24 to move a plunger mover 26 (described later).

At the other end of the guide rod 16, the plunger mover 26 to move a plunger 54 (described later) is mounted. The plunger mover 26 is provided with a pair of hooks 28a and 28b which are rotatable around a fulcrum 30. The bent ends of the hooks 28a and 28b protrude from the plunger mover 26 and have slopes. The slope formed at the bent end of the hook 28a and that of the hook 28b face to each other. The slopes of the hooks 28a and 28b are outwardly sloped in the direction of pushing the plunger 54 (i.e., to the left in Fig. 1). In Fig. 1, the distance between the outside of the slope provided at the bent end of the hook 28a and that of the hook 28b is a little longer than the diameter of a collar 56 (described later). A push spring 32 is provided between the hooks 28a and 28b, and the push spring 32 is energized so as to close the hooks 28a and 28b. In Fig. 1, the hooks 28a and 28b are in contact with the plunger mover 26, and the hook 28a and 28b will not rotate in the direction of closing the bent ends of the hooks 28a and 28b. The hooks 28a and 28b and the push spring 32 makes up a gripper 76.

The plunger mover 26 is provided with a release button 34, and the release button 34 has slopes that contact the opposite ends of the bent ends of the hooks 28a and 28b. The release button 34 makes up a manual release means to rotate the hooks 28a and 28b against the spring force of the push spring 32. Within the carriage 18, a clutch mechanism capable of disengaging the nut from the lead screw 12 is provided. Pushing the release button 34 will actuate the clutch mechanism to disengage the nut within the carriage 18 from the lead screw 12.

The plunger mover 26 is provided with a plunger grip sensor 36, which has a sensor arm 38. A plunger grip sensor chip 40 is supportedbytheplungermover26. These components including the plunger grip sensor 36 make up a plunger grip detecting means to detect whether or not the plunger 54 is gripped.

A groove 42 is formed on the top of the frame 2, and also a clamp 44 is mounted on the top of the frame 2. The clamp 44 contains pin drivers 58a and 58b, which expand and contract direct-driven pins 60a and 60b.

A flange 48 is provided at one end of a container 46 in which a liquid is contained. At the other end of the container 46, a liquid delivery portion 50 is provided, to which a liquid delivery channel 52 is connected. Further, the plunger 54 is inserted into the container 46, and the plunger 54 has a gasket 78 at one end that is close contact with the inner wall of the container 46. At the other end of the plunger 54, a collar 56 is provided.

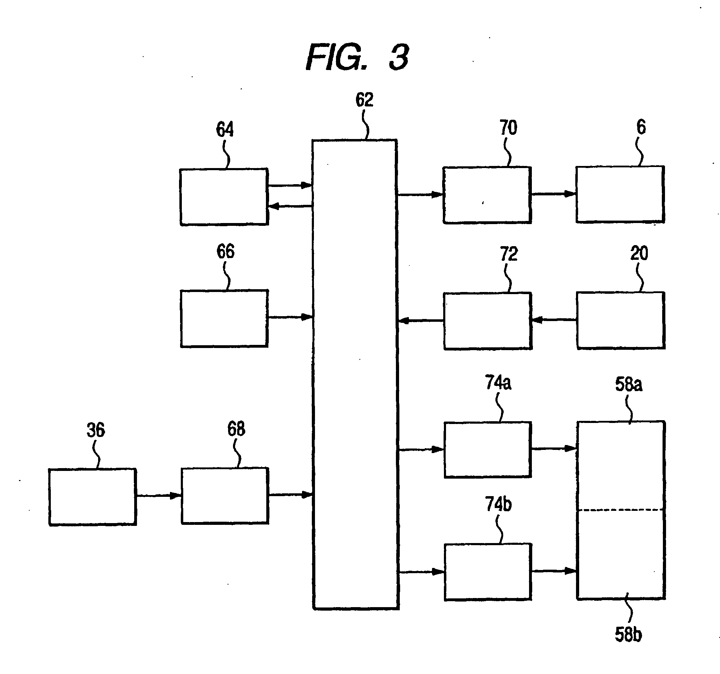

Now, referring to Fig. 3, the control device of the extrusion-type liquid delivery apparatus shown in Fig. 1 and Fig. 2 will be described. An operation/display panel 64, an initial settings input circuit 66, a sensor signal processing circuit 68, a motor drive circuit 70, a positional information processing circuit 72, and clamp drive circuits 74a and 74b are connected to a microprocessor 62. The sensor signal processing circuit 68 processes a sensor signal generated by the plunger grip sensor 36. The motor drive circuit 70 controls the drive motor 6. The positional information processing circuit 72 processes a signal from the optical sensor 20 to obtain positional information about the carriage 18. The clamp drive circuits 74a and 74b control the pin drivers 58a and 58b respectively.

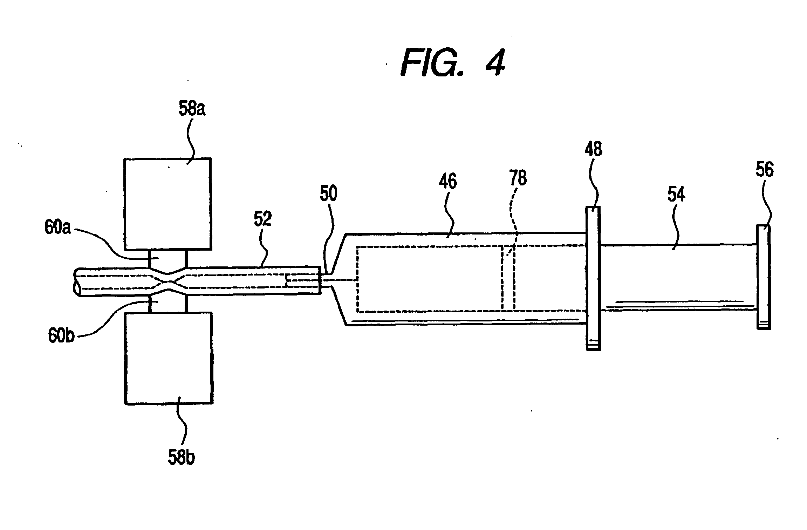

In this extrusion-type liquid delivery apparatus, when the operator engages the flange 48 into the groove 42, loads the container 46 on the top of the frame 2, and then enters a liquid delivery start command from the operation/display panel 64, the microprocessor 62 issues a clamp command to the clamp drive circuits 74a and 74b, the pin drivers 58a and 58b expand the direct-driven pins 60a and 60b respectively as shown in Fig. 4 to block the liquid delivery channel 52. If the plunger 54 is pushed through in this state, a liquidpressure in the container 46 will increase, but the liquid in the container 46 will not flow beyond the point at which the liquid delivery channel 52 is blocked.

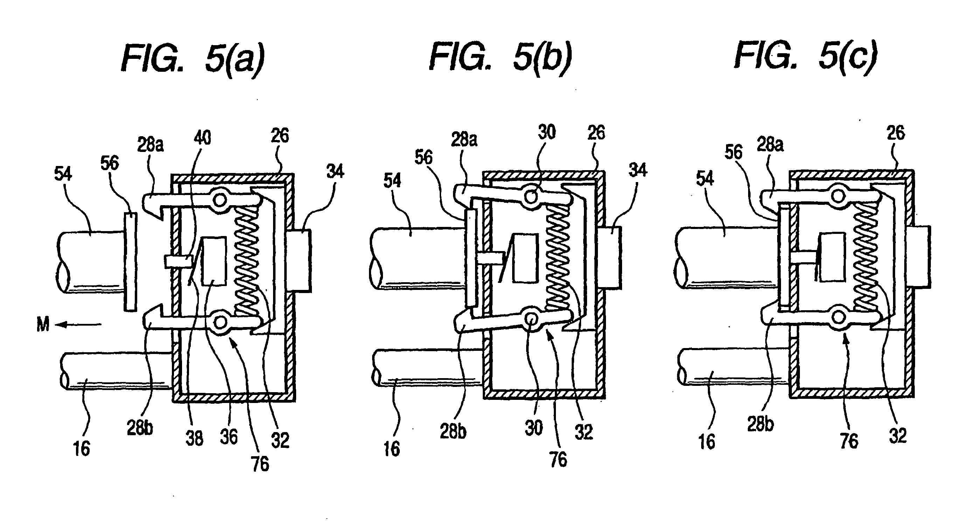

Thereafter, the microprocessor 62 issues a rotation command to the motor drive circuit 70 to rotate the drive motor 6, which moves the carriage 18 and thereby moves the plunger mover 26 in the direction of arrow M in Fig. 5 (a). At this time, the slopes formed at the bent ends of the hooks 28a and 28b contact the edge of the collar 56. When the plunger mover 26 is moved further in the direction of arrow M from this point, the hooks 28a and 28b run on the widest portion of the collar 56 against the spring force of the push spring 32, as shown in Fig. 5(b). Moving the hooks 28a and 28b further in the direction of arrow M from this state causes the hooks 28a and 28b to clime over the collar 56, and the bent ends of the hooks 28a and 28b to automatically grip the collar 56 by the force of the push spring 32 as shown in Fig. 5(c), thus completing the preparation for a liquid delivery. That is, the hooks 28a and 28b automatically rotate to grip the collar 56, simply by moving the plunger mover 26.

Meanwhile, the microprocessor 62 monitors a grip sensor signal to be input from the plunger grip sensor 36 via the sensor signal processing circuit 68, in order to determine whether the plunger 54 is gripped. Specifically, when the plunger mover 26 is moved in the direction of arrow M from the state in Fig. 5(b), the collar 56 pushes the plunger grip sensor chip 40 in the opposite direction of arrow M, and the plunger grip sensor chip 40 in turn pushes the sensor arm 38, thereby turning on the plunger grip sensor 36. Therefore, if the microprocessor 62 monitors the output of the plunger grip sensor 36, it is possible to detect whether or not the gripper 76 has gripped the collar 56, namely, the plunger 54. Also, it is possible to obtain an encoder signal from the optical sensor 20, via the positional information processing circuit 72, and to determine the current position of the carriage 18 exactly.

Then, the microprocessor 62 issues a rotation command to the motor drive circuit 70 to rotate the drive motor 6 and thereby to move the carriage 18, resulting in the plunger 26 moving, in the direction opposite to the direction of arrow M in Fig. 5(a), i.e., the direction of pushing the plunger 54, by a predetermined distance. That is, the control device moves the plunger mover 26 in the direction opposite to the direction of pushing the plunger 54 by the predetermined distance, after the hooks 28a and 28b of the gripper 76 have gripped the plunger 54. In this case, a load sensor may be provided so as to face and contact the pushed surface of the collar 56 of the plunger 54, and the microprocessor 62 may monitor the output (a blocking pressure) of the load sensor and move the plunger mover 26 in the direction opposite to the direction of pushing the plunger 54, until the output of the load sensor falls below a prescribed value.

After the plunger 54 is automatically gripped in this way, the microprocessor 62 issues a clamp release command to the clamp drive circuits 74a and 74b to cause the pin drivers 58a and 58b to contract the direct-driven pins 60a and 60b respectively as shown in Fig. 2, thereby unblocking the liquid delivery channel 52. Then, the microprocessor 62 controls the drive motor 6 via the motor drive circuit 70 to move the plunger mover 26 to the left in Fig. 1, pushing the plunger 54 into the container 46 to deliver the liquid.

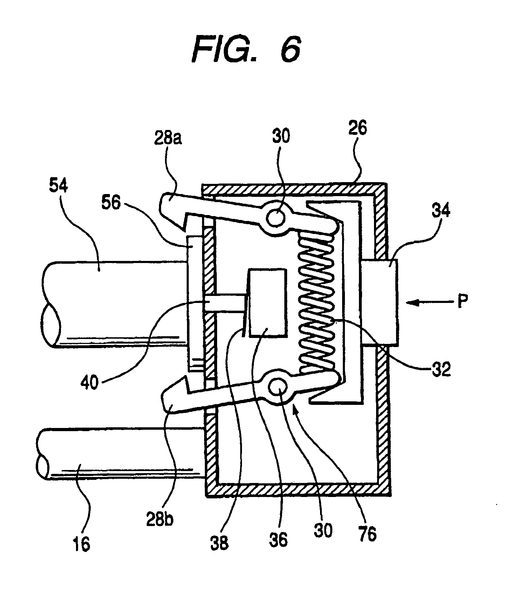

After the liquid delivery is completed, the operator manually pushes the release button 34 in the direction of arrow P as shown in Fig. 6. This produces a movement to shorten the distance between the rear ends of the hooks 28a and 28b against the force of the push spring 32, and thereby to rotate the hooks 28a and 28b around the fulcrum 30, and consequently the bent ends of the hooks 28a and 28b spread out to release the collar 56. At this time, if the release button 34 is pushed, the clutch mechanism is actuated to disengage the nut in the carriage 18 from the lead screw 12, allowing the plunger mover 26 to be moved manually. Therefore, moving the plunger mover 26 in the direction opposite to arrow P, with the release button 34 pushed in the direction of arrow P, will release the collar 56 from the hooks 28a and 28b, and also allow the plunger mover 26 to move to the point where a liquid delivery starts.

In such extrusion-type liquid delivery apparatus, the hooks 28a and 28b automatically rotate to grip the collar 56, simply by moving the plunger mover 26 and the gripper 76, and therefore the operator can perform the mounting of the container 46 containing a liquid and the gripping the plunger 54 with a plurality of liquid delivery apparatuses in a short period of time. Also, since there is no need to add a special actuator for automatic gripping, the structure is simple and manufacturing cost can be reduced. Moreover, moving the plunger mover 26 in the direction opposite to arrow P, with the release button 34 pushed in the arrow P direction, will release the collar 56 from the hooks 28a and 28b, and also allows the plunger mover 26 to move to the point where a liquid delivery starts. This makes it possible to remove an emptied container 46 and to pull the plunger mover 26 back to the prescribed position manually, and therefore the container 4 6 can be replaced quickly. Furthermore, since the output of the plunger grip sensor 36 is monitored by the microprocessor 62, it is possible to detect whether or not the gripper 76 has gripped the collar 56, namely the plunger 54, and therefore the plunger 54 can be gripped reliably by the gripper 76. Also, the plunger mover 26 is moved in the direction opposite to the direction of pushing the plunger 54 after the collar 56, namely the plunger 54, has been gripped by the gripper 76, and therefore, even if the inner pressure of the container 46 rises when the gripper grips the collar 56, the inner pressure of the container 46 can be lowered. This prevents the liquid from being delivered at a burst when the direct-driven pins 60a and 60b unblocked the liquid delivery channel 52.

For the drive motor 6, a stepping motor or DC motor can be employed. For the position detecting means to detect the position of the carriage 18, a magnetic position detector can be used. Also, an optical or magnetic rotary encoder can be provided at the end of the lead screw 12, as a position detecting means to detect the position of the carriage 18. Alternatively, a plunger mover driver having a linear motor can be used for this purpose. Although the plunger grip sensor 36 is used as a plunger grip detecting means in the above embodiment, a magnetic proximity switch or a load sensor that faces and contacts the collar 56 can be used as a plunger grip detecting means. Also, other springs can be used instead of the push spring 32 used in the above embodiment, to close the bent ends of the hooks 28a and 28b by spring force.

The extrusion-type liquid delivery apparatus according to the present invention can be used for applications that require automatic gripping of a plunger which is enabled with a simple construction, such as a medical syringe pump to deliver a liquid contained beforehand with a plunger.

## Claims

1. An extrusion-type liquid delivery apparatus, wherein a liquid in a container (46) having a liquid delivery portion (50) at one end of the container is extruded by a plunger (54) that is inserted from the other end of the container, comprising:
a) a plunger mover (26) to move said plunger;
b) a plunger mover driver (6) to move said plunger mover;
c) a pair of hooks (28a, 28b) rotatably mounted on said plunger mover with bent ends, and having slopes at said bent ends; and
d) a spring (32) to close said bent ends of said pair of hooks by spring force,
**characterised in that** the apparatus further comprises a clamp to block a liquid delivery channel connected to said container, and **in that** said bent ends project from said plunger mover, wherein, when said plunger mover moves, said slopes contact the edge of the collar of said plunger, said hooks run on the widest portion of said collar against the spring force of said spring, said hooks climb over said collar, and said bent ends automatically grip said collar by the force of said spring.

2. The extrusion-type liquid delivery apparatus of Claim 1, further comprising a manual release means (34) to rotate said hooks against the spring force of said spring.

3. The extrusion-type liquid delivery apparatus of Claim 1, further comprising a plunger grip detecting means (36) to detect whether or not said plunger is gripped.

4. The extrusion-type liquid delivery apparatus of Claim 1, further comprising a means to lower the inner pressure of said container after said hooks have gripped said plunger.

5. The extrusion-type liquid delivery apparatus of Claim 4, further comprising a control device (62) to move said plunger mover by a predetermined distance in the direction opposite to the direction of pushing said plunger, after said hooks have gripped said plunger.

## Patentansprüche

1. Flüssigkeitsausgabevorrichtung vom Extrusionstyp, wobei eine Flüssigkeit in einem Behälter (46) mit einem Flüssigkeitsausgabeabschnitt (50) an einem Ende des Behälters durch einen Kolben (54) extrudiert wird, der von dem anderen Ende des Behälters aus eingeführt wird, wobei die Vorrichtung Folgendes umfasst:
a) einen Kolbenmitnehmer (26) zur Bewegung des Kolbens;
b) einen Kolbenmitnehmerantrieb (6) zur Bewegung des Kolbenmitnehmers;
c) ein Paar Haken (28a, 28b), die drehbar mit gebogenen Enden an dem Kolbenmitnehmer angebracht sind und an den gebogenen Enden Abschrägungen aufweisen; und
d) eine Feder (32), um die gebogenen Enden des Haken-Paars mittels Federkraft zu schließen,
**dadurch gekennzeichnet, dass** die Vorrichtung ferner eine Klemme umfasst, um einen Flüssigkeitsabgabekanal, der mit dem Behälter verbunden ist, zu blockieren, und dass die gebogenen Enden von dem Kolbenmitnehmer vorstehen, wobei bei Bewegung des Kolbenmitnehmers die Abschrägungen den Rand des Bunds des Kolbens kontaktieren, wobei die Haken am breitesten Abschnitt des Bunds gegen die Federkraft der Feder auseinandergedrückt werden, und wobei sich die Haken über den Bund hinweg bewegen und wobei die gebogenen Enden den Bund durch die Kraft der Feder automatisch fassen.

2. Flüssigkeitsausgabevorrichtung vom Extrusionstyp nach Anspruch 1, ferner umfassend ein manuelles Freigabemittel (34), um die Haken gegen die Federkraft der Feder zu drehen.

3. Flüssigkeitsausgabevorrichtung vom Extrusionstyp nach Anspruch 1, ferner umfassend ein Detektionsmittel (36) für das Fassen des Kolbens, um zu detektieren, ob der Kolben erfasst ist oder nicht.

4. Flüssigkeitsausgabevorrichtung vom Extrusionstyp nach Anspruch 1, ferner umfassend ein Mittel zur Senkung des Innendrucks des Behälters, nachdem die Haken den Kolben erfasst haben.

5. Flüssigkeitsausgabevorrichtung vom Extrusionstyp nach Anspruch 4, ferner umfassend eine Steuervorrichtung (62) zur Bewegung des Kolbenmitnehmers über eine vorbestimmte Strecke hinweg in die der Druckrichtung des Kolbens entgegengesetzte Richtung, nachdem die Haken den Kolben erfasst haben.

## Revendications

1. Appareil de délivrance de liquide de type extrusion, où un liquide dans un contenant (46) ayant une portion de délivrance de liquide (50) à une extrémité du contenant est extrudé par un plongeur (54) qui est inséré depuis l'autre extrémité du contenant, comprenant:
a) un organe de déplacement de plongeur (26) pour déplacer ledit plongeur;
b) un organe d'entraînement en déplacement de plongeur (6) pour déplacer ledit organe de déplacement de plongeur;
c) deux crochets (28a, 28b) montés en rotation sur ledit organe de déplacement de plongeur avec des extrémités courbées, et ayant des inclinaisons auxdites extrémités courbées; et
d) un ressort (32) pour fermer lesdites extrémités courbées des deux crochets par une force de ressort,
**caractérisé en ce que** l'appareil comprend en outre un organe de serrage pour bloquer un canal de délivrance de liquide relié audit contenant, et **en ce que** lesdites extrémités courbées font saillie dudit organe de déplacement de plongeur où, lorsque ledit organe de déplacement de plongeur se déplace, lesdites inclinaisons viennent en contact avec le bord du collier dudit plongeur, lesdits crochets s'étendent sur la portion la plus large dudit collier contre la force dudit ressort, lesdits crochets passent par-dessus ledit collier et lesdites extrémités courbées saisissent automatique ledit collier par la force dudit ressort.

2. Appareil de délivrance de liquide de type extrusion selon la revendication 1, comprenant en outre un moyen de relâchement manuel (34) pour faire tourner lesdits crochets contre la force dudit ressort.

3. Appareil de délivrance de liquide de type extrusion selon la revendication 1, comprenant en outre un moyen de détection de prise de plongeur (36) pour détecter si oui ou non ledit plongeur est pris.

4. Appareil de délivrance de liquide de type extrusion selon la revendication 1, comprenant en outre un moyen pour diminuer la pression interne dudit contenant après que lesdits crochets ont saisi ledit plongeur.

5. Appareil de délivrance de liquide de type extrusion selon la revendication 4, comprenant en outre un dispositif de commande (62) pour déplacer ledit organe de déplacement de plongeur sur une distance prédéterminée dans la direction opposée à la direction de poussée dudit plongeur après que lesdits crochets ont saisi ledit plongeur.
